# EUROPEAN PATENT APPLICATION

(11) **EP 2 301 441 A1**
(43) Date of publication of application: **30.03.2011**
(21) Application number: 10177989.0
(22) Date of filing: 21.09.2010
(51) Int. Cl.: A61B 6/02

(54) **Radiation imaging apparatus**

(30) Priority: 24.09.2009 JP 2009218745
(71) Applicant: FUJIFILM Corporation, Tokyo (JP)
(72) Inventor: Takahashi, Shoji, Kanagawa-ken (JP)
(74) Representative: Klunker . Schmitt-Nilson . Hirsch

(57) **Abstract**

A radiation imaging apparatus capable of easily improving measurement accuracy of the position of tube focus in radiation imaging. The apparatus includes a radiation source (30) for emitting radiation, a radiation detector (20) for detecting radiation emitted from the radiation source (30) and transmitted through a subject (1A), a shifting section (10) for holding and shifting the radiation source (30) in a direction parallel to a detection surface (20M) of the radiation detector (20), and an acceleration sensor (40S) for obtaining a position of a tube focus (30F) of the radiation source (30), in which the acceleration sensor (40S) is integrally disposed adjacent to the radiation source (30) and radiation imaging is performed while shifting the radiation source (30) by the shifting section (10).

## Description

### BACKGROUND OF THE INVENTION

### Field of the Invention

The present invention relates to a radiation imaging apparatus, and more particularly to a radiation imaging apparatus for obtaining a plurality of radiation images used for generating a tomosynthesis image.

### Description of the Related Art

Tomosynthesis imaging in which radiation imaging is performed a plurality of times by shifting a radiation source with respect to an examination region so that radiation is incident on the examination region at different incident angles is known as described, for example, Japanese Unexamined Patent Publication No. 63(1988)-230152.

A method in which a plurality of radiation images obtained by the tomosynthesis imaging is combined, thereby obtaining a radiation tomographic image representing cross-sections of the examination region, i.e., a tomosynthesis image is also known as described, for example, in "The principle and clinical application of Tomosynthesis", T. Shiomi, Journal of Japan Society Medical Imaging and Information Science, Vol. 24, No.2, pp. 22-27, 2007. The method obtains a tomosynthesis image by combining a plurality of radiation images such that the contrast of a predetermined cross-section of the examination region is enhanced and blur of images representing cross-sections other than the predetermined cross-section is increased.

As a system that performs such tomosynthesis imaging, a general purpose radiation imaging system in which a radiation source supported by an overhead traveling suspension that travels on a rail which is installed on a ceiling is shifted in horizontal and vertical directions is known as described, for example, in U.S. Patent No. 6,970,531 and Japanese Unexamined Patent Publication No. 2007-244569.

The general purpose radiation imaging system allows both tomosynthesis imaging by performing radiation imaging a plurality of times while shifting the radiation source by the overhead traveling suspension and general radiation imaging for obtaining a radiation image by fixing the position of the radiation source by the overhead traveling suspension and emitting radiation to an examination region once.

In comparatively small medical facilities, such as private practitioners, clinics, and the like, it is rare to have a dedicated radiation imaging system for tomosynthesis imaging and it is common to perform various types of radiation imaging using a general purpose radiation imaging system.

When performing tomosynthesis imaging using such a general purpose radiation imaging system, radiation imaging of an examination region (e.g., a spine) is performed a plurality of times by shifting the radiation source in one direction parallel to a detection surface of a radiation detector and changing the incident angle of the radiation with respect to the examination region. Thereafter, a tomosynthesis image representing a cross-section of the examination region is obtained through image synthesis using image data representing a plurality of images obtained by the tomosynthesis imaging and position data representing the position of the tube focus at each radiation imaging.

The position of the tube focus in each radiation imaging obtained in tomosynthesis imaging using such a general purpose radiation imaging system is measured by a position measuring device (e.g., linear scale measuring device) provided adjacent to the ceiling where the overhead traveling suspension is installed.

The measurement of the position of the tube focus by such a position measuring device provided adjacent to the ceiling has a large error in the position data of the tube focus because the measurement axis is located near the ceiling while the tube focus, the measurement target, is away from the ceiling (e.g., 1.5 m).

That is, when position data obtained by measuring a shift of the overhead traveling suspension by a linear scale measuring device provided along a ceiling are used as the position data of a tube focus away from the ceiling, the influence of shift errors of the overhead traveling suspension (errors of pitching, yawing, rolling, and the like) is increased such that the greater the distance of the tube focus from the ceiling, the greater the influence. Due to the so-called Abbe principle, the measurement error increases with the increase in the distance between the measurement axis (measurement axis of the linear scale measuring device) and measurement target (tube focus).

Consequently, the error when combining a plurality of radiation images obtained by the tomosynthesis imaging is also increased, posing a problem that the resolution or contrast of the tomosynthesis image obtained is degraded.

When measuring the position of the tube focus, it is preferable that the tube focus is positioned on the measurement axis for the reason described above. There is a problem here that it is difficult to construct a radiation imaging system such that the measurement axis of a position measuring device is located adjacent to the tube focus (measurement target point) of a radiation source held by an overhead traveling suspension that travels on a rail installed on a ceiling.

The problem described above is not limited to radiation imaging systems that implement tomosynsthesis imaging but is common to radiation imaging systems that perform radiation imaging while shifting the radiation source in a direction parallel to the detection surface of the radiation detector.

The present invention is developed in view of the circumstances described above and it is an object of the present invention to provide a radiation imaging apparatus capable of easily improving measurement accuracy of the position of tube focus in radiation imaging.

### SUMMARY OF THE INVENTION

A radiation imaging apparatus of the present invention is an apparatus which includes a radiation source for emitting radiation, a radiation detector for detecting radiation emitted from the radiation source and transmitted through a subject, a shifting means for holding and shifting the radiation source in a direction parallel to a detection surface of the radiation detector, and a position obtaining means for obtaining a position of a tube focus of the radiation source, and which performs tomosynthesis imaging while shifting the radiation source by the shifting means,
wherein the position obtaining means comprises an acceleration sensor integrally disposed adjacent to the radiation source.

The position of the tube focus is a position of a focus of radiation emitted from the radiation source. That is, it is a position of a focus of a tube provided in the radiation source.

The acceleration sensor integrally disposed adjacent to the radiation source is a sensor disposed adjacent to the radiation source and disposed integrally with the radiation source.

Preferably, the acceleration sensor includes a pair of sensors disposed on a straight line passing through the tube focus so as to sandwich the tube focus.

The shifting means may be a means that includes an orthogonal shifting section for shifting the radiation source in a direction orthogonal to the detection surface.

The radiation source may be a source held by an overhead traveling suspension shifting platform.

Another radiation imaging apparatus of the present invention is an apparatus which includes a radiation source for emitting radiation, a radiation detector for detecting radiation emitted from the radiation source and transmitted through a subject, a shifting means for holding and shifting the radiation source in a direction parallel to a detection surface of the radiation detector, and a position obtaining means for obtaining a position of a tube focus of the radiation source, and which performs radiation imaging while shifting the radiation source by the shifting means,
wherein the position obtaining means comprises a laser length measuring device integrally disposed adjacent to the radiation source.

The radiation imaging described above may be tomosynthesis imaging. In the tomosynthesis imaging, radiation imaging is performed a plurality of times while shifting the radiation source by the shifting means to obtain a plurality of radiation images for use in generating a tomosynthesis image representing a cross-section of a subject and the position of the tube focus at each of the plurality of times of radiation imaging is obtained.

The acceleration sensor is a sensor for measuring the position of tube focus in a three-dimensional space.

According to the radiation imaging apparatus of the present invention, the position obtaining means includes an acceleration sensor disposed adjacent to and integrally with the radiation source and the position of the tube focus is measured using the acceleration sensor. This may easily improve measurement accuracy of the position of tube focus obtained at each radiation imaging.

That is, for example, the acceleration sensor can be easily attached to a radiation source held on the lower side of an overhead traveling suspension and can be shifted in horizontal and vertical directions, because the acceleration sensor is capable of measuring acceleration by itself. Further, the position of acceleration sensor may be obtained by integrating acceleration values measured by the acceleration sensor.

This reduces the distance from the measurement axis of the acceleration sensor (position measuring device) to the tube focus (measurement target point) and allows measurements in accordance with the Abbe principle, whereby measurement accuracy of the position of tube focus may be improved.

In contrast, a linear scale measuring device, for example, is a device that performs measurement with a scale fixed on a fixed portion and a reader, provided on a moving portion, for reading a scale mark of the scale. Therefore, it is difficult to bring the measurement axis, which is defined by the combination of the fixed and moving portions, near the tube focus.

Further, if the acceleration sensor includes a pair of sensors disposed on a straight line passing through the tube focus so as to sandwich the tube focus, errors that occur according to the distance from the measurement axis to the tube focus (errors due to measurements not in accordance with the Abbe principle) may further be reduced. That is, if the distance from the tube focus to each acceleration sensor disposed opposite to each other across the tube focus is known, an error in the measurement performed by the acceleration sensor disposed on one of the sides sandwiching the tube focus can be offset by an error in the measurement performed by the acceleration sensor on the other side, whereby measurement accuracy of the position of tube focus may further be improved.

Still further, if the shifting means is a means that includes an orthogonal shifting section for shifting the radiation source in a direction orthogonal to the detection surface, or if the radiation source is a source held by an overhead traveling suspension shifting platform, measuring accuracy of the position of tube focus may be improved significantly.

That is, the magnitude of a measurement error that occurs when measuring the position of tube focus by the acceleration sensor disposed on the radiation source is mainly dependent on the distance from the position of tube focus to the acceleration sensor, so that the position of tube focus may be measured without being affected by a shift error that occurs when the radiation is shifted by the shifting means. Consequently, if radiation imaging is performed while shifting the radiation source by a shifting means that causes a relatively large error, a significant effect of improving measurement accuracy of the position of tube focus may be obtained.

### BRIEF DESCRIPTION OF THE DRAWINGS

Figure 1 is a conceptual drawing illustrating a schematic configuration of a radiation imaging apparatus according to an embodiment of the present invention.
Figure 2 illustrates radiation imaging performed in standing position using the radiation imaging apparatus shown in Figure 1.
Figure 3 is an enlarged perspective view of a shift assistance section.
Figure 4 illustrates synthesis of a plurality of images obtained by tomosynthesis imaging.
Figure 5 illustrates a positional relationship between each section when tomosynthesis imaging is performed.
Figure 6 illustrates a tolerance when tomosynthesis imaging is performed with the positional relationship shown in Figure 5.
Figure 7 illustrates deflection of a movable rail extending in an X direction.
Figure 8 shows acceleration values measured by an acceleration sensor.
Figure 9 illustrates speeds obtained by integrating the acceleration values once measured by the acceleration sensor.
Figure 10 illustrates values of speeds obtained by integrating the acceleration values twice measured by the acceleration sensor.
Figure 11 shows position errors in a range in which a horizontal shifting platform is shifted at a constant speed.
Figure 12A is a flowchart of an acceleration data measurement.
Figure 12B is a flowchart of tomosynthesis imaging.
Figure 13 illustrates radiation imaging apparatus that uses a laser length measuring device for tube position measurement.

### DESCRIPTION OF THE PREFERRED EMBODIMENTS

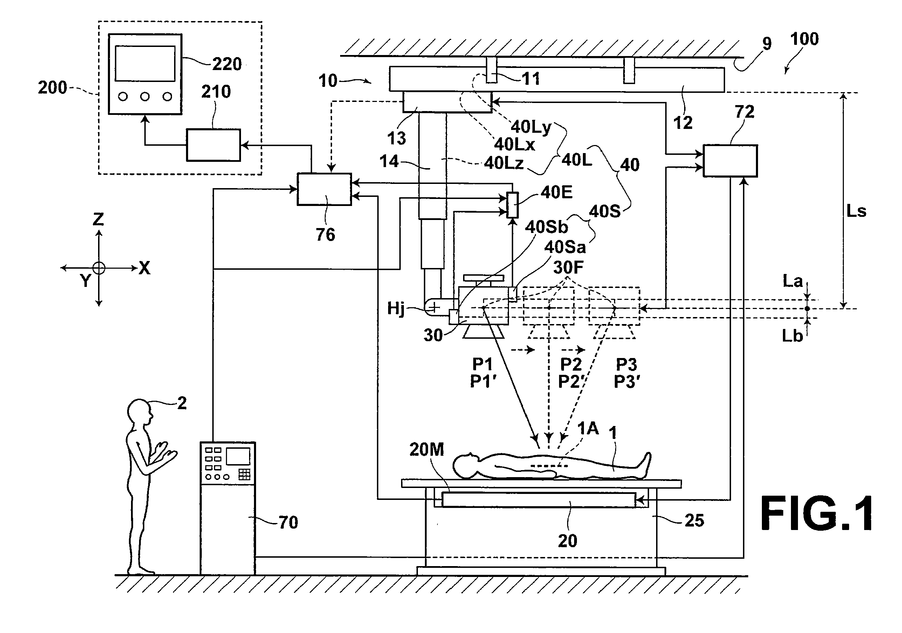

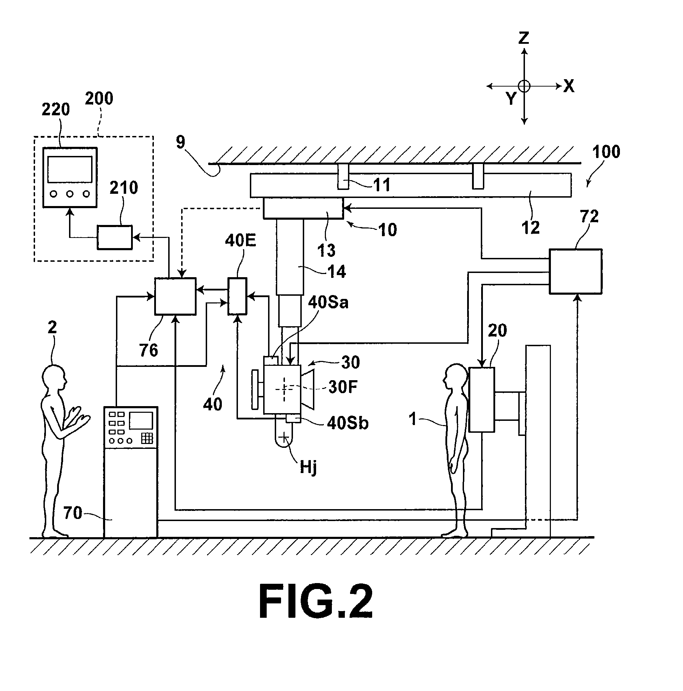

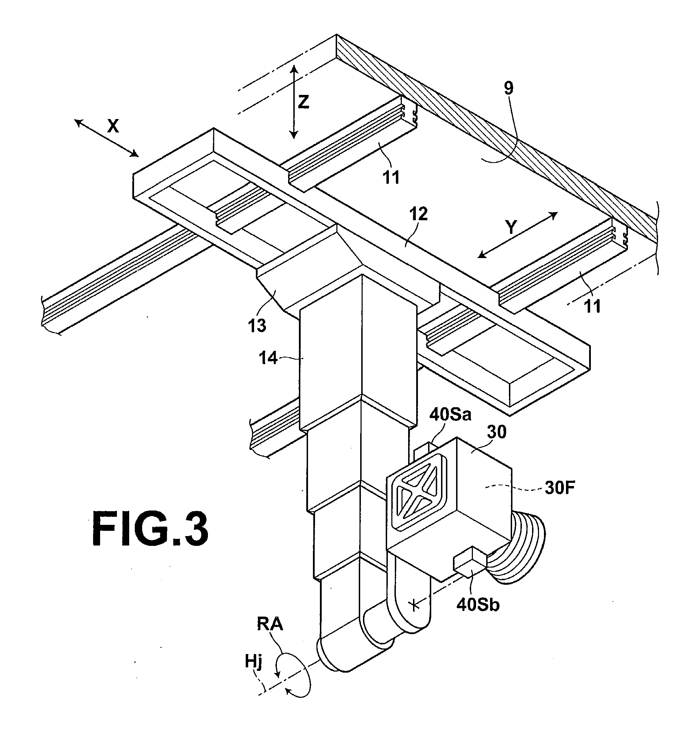

Hereinafter, embodiments of the present invention will be described with reference to the accompanying drawings. Figures 1 and 2 illustrate schematic configurations of a radiation imaging apparatus according to embodiments of the present invention. Figure 1 is a conceptual drawing illustrating radiation imaging performed in spine position. Figure 2 is a conceptual drawing illustrating radiation imaging performed in standing position. Figure 3 is an enlarged perspective view of a shift assistance section.

As shown in Figure 1, radiation imaging apparatus 100 of the present invention installed in an examination room is a general purpose radiation imaging apparatus 100. It includes radiation source 30 for emitting radiation, radiation detector 20 for detecting radiation emitted from radiation source 30 and transmitted through examination region 1A of subject 1, shifting section 10 for holding and shifting radiation source 30 in one direction parallel to detection surface 20M of radiation detector 20(X direction, here), and position obtaining section 40 for obtaining the position (in X, Y, and Z directions in Figure 1) of tube focus 30F of radiation source 30 shifted by shifting section 10. It performs tomosynthesis imaging while shifting radiation source 30 by shifting section 10.

Shifting section 10 can be shifted on a rail installed on the ceiling of the examination room, as described later.

Radiation detector 20 detects radiation transmitted through examination region 1A and representing examination region 1A.

Radiation imaging apparatus 100 performs radiation imaging of examination region 1A a plurality of times by emitting radiation from radiation source 30 while shifting radiation source 30 by shifting section 10. Through the plurality of times of radiation imaging, image data representing a plurality of radiation images used for generating a tomosynthesis image representing a cross-section of examination region 1A are obtained via radiation detector 20.

Position obtaining section 40 includes acceleration sensors 40Sa and 40Sb (hereinafter, also collectively referred to as acceleration sensors 40S), which are motion sensors integrally provided adjacent to radiation source 30 for measuring acceleration, and measures the position of tube focus 30F using acceleration sensors 40Sa and 40Sb.

Acceleration sensor 40Sa is a sensor capable of measuring acceleration in three axis directions of X, Y, and Z. Also, acceleration sensor 40Sb is a sensor capable of measuring acceleration in the three axis directions.

Position obtaining section 40 includes calculation section 40E that obtains amounts of positional displacement of acceleration sensors 40Sa and 40Sb by time-integrating acceleration values obtained by respective acceleration sensors 40Sa and 40Sb.

Acceleration sensors 40Sa and 40Sb are disposed on a straight line passing through tube focus 30F so as to sandwich tube focus 30F, i.e., each on each side of tube focus 30F.

Here, acceleration sensors 40Sa and 40Sb are equidistant from tube focus 30F, so that calculation section 40E may obtain the amount of positional displacement of tube focus 30F by averaging each set of coordinates, obtained by calculation section 40E, representing the amount of positional displacement of each of acceleration sensors 40Sa and 40Sb.

Distance La from tube focus 30F (measurement target point) to acceleration sensor 40Sa (measurement axis) in Z axis direction and distance Lb from tube focus 30F (measurement target point) to acceleration sensor 40Sb (measurement axis) in Z axis direction are equal and they may be, for example, 0.1 m.

Note that even in a case where acceleration sensors 40Sa and 40Sb are not equidistant from tube focus 30F, if the ratio of the distance from tube focus 30F to acceleration sensor 40Sa and the distance from tube focus 30F to acceleration sensor 40Sb is known, the amount of positional displacement of tube focus 30F may be obtained by a known method as described above.

Calculation section 40E adds the amount of positional displacement obtained in the manner described above to predetermined and inputted coordinates representing the reference position of tube focus 30F and outputs coordinate data representing the position of tube focus 30F.

More specifically, radiation source 30 is disposed such that tube focus 30F is placed at a predetermined reference position (e.g., coordinates (0, 0, 0)) in the examination room. Then, the position of tube focus 30F may be obtained by adding the amount of displacement obtained by calculation section 40E to the coordinates (0, 0, 0) of the reference position. This allows, when, for example, the position coordinates of examination table 72 with respect to the reference position, the position coordinates of examination region 1A with respect to the reference position, or the like is obtained and stored in calculation section 40E in advance, the position of tube focus 30F with respect to these positions described above may be obtained. In this way, position obtaining section 40 may obtain the position of tube focus 30F with respect to an object which is stationary relative to the examination room.

Here, it is assumed that position obtaining section 40 obtains coordinate data representing the position of tube focus 30F in three axis directions of one direction parallel to detection surface 20M (arrow X directions in Figure 1), a direction orthogonal to the one direction and parallel to detection surface 20M (arrow Y directions in Figure 1), and a direction orthogonal to detection surface 20M (arrow Z directions in Figure 1).

As shown in Figure 1, shifting section 10 includes fixed rails 11 installed on ceiling 9, movable rail 12 extending in a direction (an X direction) orthogonal to the extending direction of fixed rails 11 (a Y direction), which is suspended by fixed rails 11 and movable in the extending direction of fixed rails 11 (a Y direction), horizontal shifting platform 13 which is suspended by movable rail 12 and movable in the extending direction of movable rail 12 (X direction), extensible/retractable support 14 which is attached to the underside of horizontal shifting platform 13 and is extensible/retractable in a vertical direction (a Z direction) by a multistage extension mechanism, drive motors (not shown) for shifting horizontal shifting platform 13 in X and Y directions and extending/retracting extendable/retractable support 14 in a Z direction.

Radiation source 30 is attached to a second end of extendable/retractable support 14, a first end of which is fixed to horizontal shifting platform 13.

This allows shifting section 10 to shift radiation source 30 in two mutually orthogonal directions (X and Y directions) which are parallel to detection surface 20M and an orthogonal direction (a Z direction) orthogonal to detection surface 20M.

Radiation source 30 attached to the second end of extendable/retractable support 14 is rotatably supported about horizontal axis Hj (arrow RA directions in Figure 3) so as to be changeable in position with respect to extendable/retractable support 14, thereby allowing radiation imaging in spine position (Figure 1) and radiation imaging in standing position (Figures 2 and 3).

In addition to acceleration sensors 40S, position obtaining section 40 includes linear scale measuring devices 40L.

Linear scale measuring device 40 includes linear scale measuring device 40Ly for Y axis constituted by a linear scale fixed along fixed rail 11 which is installed on the ceiling and extending in a Y direction and a reading section, fixed to movable rail 12, for reading a scale mark of the linear scale. Linear scale measuring device 40 also includes linear scale measuring device 40Lx for position measurement in an X axis direction constituted by a linear scale fixed along movable rail 12 which is installed on the ceiling and a reading section, fixed to horizontal shifting platform 13, for reading a scale mark of the linear scale. Linear scale measuring device 40 further includes linear scale measuring device 40Lz for measuring the position of the tip of extendable/retractable support 14 in a Z axis direction. Note that these linear scales are omitted in Figure 1.

Linear scale measuring devices 40Lx and 40Ly may accurately measure positions of horizontal shifting platform 13 in X and Y directions since horizontal shifting platform 13, which is the measurement target point, is located adjacent to the measurement axes thereof. When the measurement target point is tube focus 30F, however, the measurement axes of linear scale measuring devices 40Lx and 40Ly are far away from the measurement target point. Distance Ls in a Z axis direction from tube focus 30F (measurement target point) to the measurement axis of linear scale measuring device 40Lx is 1.5 m.

Consequently, measurement error is larger when the position of tube focus 30F in X and Y directions is obtained by linear scale measuring devices 40Lx and 40Ly than in the case in which the position of horizontal shifting platform 13 in X and Y directions is measured by linear scale measuring devices 40Lx and 40Ly due to the so-called Abbe principle.

Including such error, the measurement of the shifting of tube focus 30F in X, Y, and Z directions by linear scale measuring devices 40Lx, 40Ly, 40Lz (also, collectively referred to as linear scale measuring devices 40L) will be described later.

Note that acceleration sensors 40Sa and 40Sb are located adjacent to the measurement target point of tube focus 30F and each of distances La and Lb from tube focus 30F (measurement target point) to acceleration sensors 40Sa and 40Sb in a Z axis direction is 0.1 m, while distance Ls from tube focus 30F (measurement target point) to the measurement axis of linear scale measuring device 40Lx is 1.5 m.

Therefore, when the position of tube focus 30F is measured using acceleration sensors 40Sa and 40Sb, measurement errors included in measurement values arising from the influence of shifting errors of shifting section 10 (pitching, rolling, yawing) are reduced to 1/15 of measurement errors included in measurement values arising from the influence of shifting errors of shifting section 10 (pitching, rolling, yawing) when tube focus 30F is measured by linear scale measuring device 40Lx.

Further, calculation section 40E may perform calculation such that an error (Abbe principle error) occurred in the position measurement of tube focus 30F by acceleration sensor 40Sa is offset by an error (Abbe principle error) occurred in the position measurement of tube focus 30F by acceleration sensor 40Sb. This allows position obtaining section 40 to accurately measure the position of tube focus 30F.

Image data obtained by radiation detector 20 and position data obtained by position obtaining section 40 of radiation imaging apparatus 100 are temporarily stored in storage section 76 and then inputted to tomosynthesis image generation section 210 of radiation image reproducing apparatus 200. Then, tomosynthesis image generation section 210 generates a tomosynthesis image using the position data obtained by position obtaining section 40 and image data representing a plurality of radiation images obtained by radiation detector 20. Note that the position of examination region 1A of subject 1 on imaging platform 25 measured separately is inputted to tomosynthesis image generation section 210 via console 70, to be described later, and storage section 76.

The operation, synchronization of operational timing, and the like of each section of radiation imaging apparatus 100 that performs tomosynthesis imaging for obtaining image data and position data used for generating a tomosynthesis image are controlled by controller 72. Controller 72 controls each section according to an instruction inputted from console 70.

An operation of radiation imaging apparatus 100 when performing tomosynthesis imaging will now be described.

First, a case in which the position of tube focus 30F is measured using only acceleration sensors 40Sa and 40Sb will be described. Then, a case in which the position of tube focus 30F is measured using only linear scale measuring devices 40L will be described. Finally, a case in which the position of tube focus 30F is measured in combination with acceleration sensors 40S and linear scale measuring devices 40L will be described.

### <Case in which Position of Tube Focus is Measured Using Only Acceleration Sensors>

When performing tomosynthesis imaging in spine position, radiation detector 20 is accommodated in spine position imaging platform 25, and subject 1 is placed in spine position on the upper side of imaging platform 25, i.e., on the side of detection surface 20M of radiation detector 20 accommodated in spine position imaging platform 25 (Figure 1).

Then, operator 2 enters position data obtained by measuring the position of examination region 1A of subject 1 in spine position, imaging conditions of tomosynthesis imaging, and the like in console 70.

Next, operator 2 operates console 70 to shift radiation source 30 such that tube focus 30F is placed at the reference position of coordinates (0, 0, 0). The reference position of coordinates (0, 0, 0) may be, for example, a position of tube focus 30F when radiation source 30 is abutted to a predetermined abutment surface.

Then, operator 2 operates console 70 to start tomosynthesis imaging. This causes an instruction to perform predetermined tomosynthesis imaging to be inputted to controller 72 from console 70. Controller 72 controls each section so that tomosynthesis imaging for obtaining a tomographic image (tomosynthesis image) of examination region 1A of subject 1 is performed.

First, radiation source 30 is shifted through control of controller 72 such that tube focus 30F located at the reference position of coordinates (0, 0, 0) is placed at the initial position of coordinates (X₀, Y₀, Z₀). That is, radiation source 30 is shifted such that the position of tube focus 30F obtained by position obtaining section through measurement using only acceleration sensors 40S corresponds to coordinates (X₀, Y₀, Z₀) (Figure 1).

) Thereafter, tomosythesis imaging by radiation imaging apparatus 100 is started.

As shown in Figure 1, horizontal shifting platform 13 is shifted by shifting section 10 along movable rail 12 extending in an X direction. This causes radiation source 30, attached to the tip of extensible/retractable support 14, to be shifted also in the X direction.

Here, the shifting section 10 shifts horizontal shifting platform 13 only on movable rail 12 extending in an X direction without extending/retracting extensible/retractable support 14 extending in a Z direction and without shifting horizontal shifting platform 13 on fixed rails 11 extending in a Y direction.

While being shifted in the X direction which is one direction parallel to detection surface 20M of radiation detector 20, radiation source 30 emits radiation onto examination region 1A at first imaging position P1. First imaging position P1 is a position where X coordinate of the position of tube focus 30F obtained by position obtaining section 40 is X1. The position of tube focus 30F obtained by position obtaining section 40 at first imaging position P1 corresponds to coordinates (X1, Y1, Z1).

Here, if shifting section 10 could shift radiation source 30 in one direction (X direction) without any error, the position of tube focus 30F obtained by position obtaining section 40 would be coordinates (X1, Y₀, Z₀). But, tube focus 30F is shifted with slightly being displaced in Y and Z directions. Consequently, when tube focus 30F arrives at first imaging position P1 (position where the coordinate in X direction obtained by position obtaining section 40 is X1), the position of tube focus 30F is displaced in a Y direction (amount of displacement (Y1-Y₀)) and a Z direction (amount of displacement (Z1-Z₀)), so that the position of tube focus 30F is obtained as coordinates (X1, Y1, Z1).

That is, due to deformation of movable rail 12 extending in an X direction along the ceiling, deformation of ceiling itself, or the like, horizontal shifting platform 13 shifted on movable rail 12 in the X direction is displaced in Y and Z directions or slightly rotated about X axis, Y axis, and Z axis. That is, along with the shift of horizontal shifting platform 13, pitching, rolling, and yawing occur in horizontal shifting platform 13.

Then, while being shifted in the X direction, radiation source 30 emits radiation toward examination region 1A at second imaging position P2. Second imaging position P2 is a position where X coordinate of the position of tube focus 30F obtained by position obtaining section 40 is predetermined coordinate X2. The position of tube focus 30F obtained by position obtaining section 40 at second imaging position P2 corresponds to coordinates (X2, Y2, Z2).

Here, if shifting section 10 could shift radiation source 30 in one direction (X direction) without any error, the position of tube focus 30F obtained by position obtaining section 40 at second imaging position P2 would be coordinates (X2, Y₀, Z₀). But, as in the above, when tube focus 30F arrives at second imaging position P2 (position where the coordinate in X direction obtained by position obtaining section 40 is X2), the position of tube focus 30F is displaced in a Y direction (amount of displacement (Y2-Y₀)) and a Z direction (amount of displacement (Z2-Z₀)), so that the position of tube focus 30F is obtained as coordinates (X2, Y2, Z2).

Then, while being shifted in the X direction, radiation source 30 emits radiation toward examination region 1A at third imaging position P3. Third imaging position P3 is a position where X coordinate of the position of tube focus 30F obtained by position obtaining section 40 is predetermined coordinate X3. The position of tube focus 30F obtained by position obtaining section 40 at third imaging position P3 corresponds to coordinates (X3, Y3, Z3).

Here, if shifting section 10 could shift radiation source 30 in one direction (X direction) without any error, the position of tube focus 30F obtained by position obtaining section 40 at third imaging position P3 would be coordinates (X3, Y₀, Z₀). But, as in the above, when tube focus 30F arrives at third imaging position P3 (position where the coordinate in X direction obtained by position obtaining section 40 is X3), the position of tube focus 30F is displaced in a Y direction (amount of displacement (Y3-Y₀)) and a Z direction (amount of displacement (Z3-Z₀)), so that the position of tube focus 30F is obtained as coordinates (X3, Y3, Z3).

Through the description above, image data representing a radiation image taken at each of first to third imaging positions P1 to P3 are obtained via radiation detector 20 and position data representing the position of tube focus 30F at each of imaging positions P1 to P3 are obtained by position obtaining section 40, whereby the tomosynthesis imaging is completed.

The position data obtained by position obtaining section 40 and the image data representing a plurality of radiation images obtained via radiation detector 20 in the tomosynthesis imaging described above are temporarily stored in storage section 76 and then inputted to tomosynthesis image generation section 210. Thereafter, tomosynthesis image generation section 210 generates a tomosynthesis image representing a cross-section of examination region 1A by reconstructing the image data using the inputted position data.

The tomosynthesis image generated in the manner described above is displayed on display device 220 of radiation image reproducing apparatus 200.

A method of obtaining a tomosynthesis image by combining a plurality of radiation images obtained by the tomosynthesis imaging is described in detail, for example, in "The principle and clinical application of Tomosynthesis", T. Shiomi, Journal of Japan Society Medical Imaging and Information Science, Vol. 24, No.2, pp. 22-27, 2007.

### <Case in which Position of Tube Focus is Measured Using Only Linear Scale Measuring Devices>

Next, a case in which the position of tube focus is measured using only the linear scale measuring devices will be described.

Steps to the start of tomosynthesis imaging by radiation imaging apparatus 100 are identical to those of the case in which the imaging is performed using only the acceleration sensors and, therefore, they will not be elaborated upon further here. Further, in the steps that follow, only the measuring method of the position of tube focus 30 is different, so that the measurement using only the linear scale measuring devices will be described with reference to Figure 1.

As illustrated in Figure 1, when tomosynthesis imaging by radiation imaging apparatus 100 is started, horizontal shifting platform 13 is shifted by shifting section 10 along movable rail 12 extending in an X direction. This causes radiation source 30, attached to the tip of extensible/retractable support 14, to be shifted also in the X direction, whereby the tube focus is also shifted in the X direction.

Here, the shifting section 10 shifts horizontal shifting platform 13 only on movable rail 12 extending in an X direction without extending/retracting extensible/retractable support 14 extending in a Z direction and without shifting horizontal shifting platform 13 on fixed rails 11 extending in a Y direction.

While being shifted in the X direction which is one direction parallel to detection surface 20M of radiation detector 20, radiation source 30 emits radiation onto examination region 1A at first imaging position P1'. First imaging position P1' is a position where X coordinate of the position of tube focus 30F obtained by position obtaining section 40 using the linear scale measuring devices is X1. The position of tube focus 30F obtained by position obtaining section 40 at first imaging position P1' corresponds to coordinates (X1, Y₀, Z₀).

Here, shifting section 10 does not shift radiation source 30 in the X direction without any error, but horizontal shifting platform 13 shifted on movable rail 12 in the X direction is displaced in Y and Z directions or slightly rotated about X axis, Y axis, and Z axis (pitching, rolling, yawing). The position of tube focus 30F varies with such shifting errors.

Consequently, the position of tube focus 30F is displaced, in actuality, from the position represented by the coordinates obtained by position obtaining section 40. Such shifting errors, however, are not detected when the measurement is performed using only the linear scale measuring devices attached in the manner described above.

That is, shifting errors occurred when horizontal shifting platform 13 arrives at first imaging position P1' (position where the coordinate in the X direction obtained by linear scale measuring device 40Lx is X1) and first imaging is performed, so that the position of first imaging position P1' obtained by position obtaining section 40 corresponds to coordinates (X1, Y₀, Z₀).

Note that, the coordinate value in the X direction of first imaging position P1' obtained by position obtaining section 40 is X1, but the value X1 also includes a measurement error due to a measurement not in accordance with the Abbe principle.

Then, when radiation source 30 is shifted in the X direction and arrives at second imaging position P2' (position where the coordinate in the X direction obtained by linear scale measuring device 40Lx of position obtaining section 40 is X2), second imaging is performed. Since shifting errors described above are not detected, the position of second imaging position P2' obtained by position obtaining section 40 corresponds to coordinates (X2, Y₀, Z₀).

Then, when radiation source 30 is shifted in the X direction and arrives at third imaging position P3' (position where the coordinate in the X direction obtained by linear scale measuring device 40Lx of position obtaining section 40 is X3), third imaging is performed. Here also, shifting errors described above are not detected, so that the position of third imaging position P2' obtained by position obtaining section 40 corresponds to coordinates (X3, Y₀, Z₀).

As described above, the positions of tube focus 30F at first imaging position P1', second imaging position P2', and third imaging position P3' are obtained by position obtaining section as coordinates (X1, Y₀, Z₀), coordinates (X2, Y₀, Z₀), and coordinates (X3, Y₀, Z₀) respectively. These coordinates, however, include errors due to a measurement not in accordance with the Abbe principle.

Through the description above, image data representing a radiation image taken at each of first to third imaging positions P1' to P3' are obtained via radiation detector 20 and position data (position data measured using only the linear scale measuring device) representing the position of tube focus 30F at each of imaging positions P1' to P3' are obtained by position obtaining section 40, whereby the tomosynthesis imaging is completed.

The image data and position data obtained by the tomosynthesis imaging described above are temporarily stored in storage section 76 and then inputted to tomosynthesis image generation section 210. Thereafter, tomosynthesis image generation section 210 generates a tomosynthesis image representing a cross-section of examination region 1A by reconstructing the image data using the inputted position data. The tomosynthesis image generated in the manner described above is displayed on display device 220.

### <Case in which Position of Tube Focus is Measured in Combination With Acceleration sensors 40S and Linear Scale Measuring Devices 40L>

When measuring the position of tube focus in combination with acceleration sensors 40S and linear scale measuring devices 40L, it is necessary to combine an acceleration sensor and linear scale measuring device to be actually used in consideration of the performance thereof. For example, when measuring the position in an X direction, an arrangement may be adopted in which measurement result of linear scale measuring device 40Lx is corrected by measurement result of acceleration sensors 40S.

### <Generation of Tomosynthesis Image>

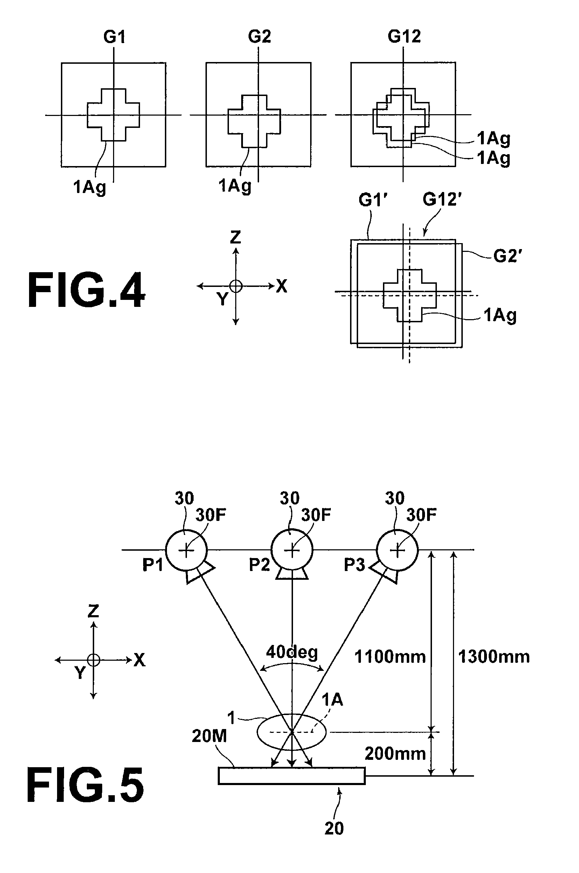

Figure 4 illustrates combining of a plurality of images obtained by tomosythesis imaging.

For example, if image G1 obtained by the imaging at first imaging position P1 and image G2 obtained by the imaging at second imaging position P2 are simply superimposed on top of each other to combine them without correcting the positional relationship, images 1Ag representing examination region 1A are misaligned and superimposed, as superimposed image G12.

In such a case, a correction is performed on images G1 and G2 using coordinates (X1, Y1, Z1) and (X2, Y2, Z2) respectively representing first imaging position P1 and second imaging position P2 obtained by the measurement of acceleration sensors 40S. That is, the correction is performed such that image G1 and image G2 are obtained at predetermined positions of coordinates (X1, Y₀, Z₀) and coordinates (X2, Y₀, Z₀) respectively. That is, in tomosynthesis image generation section 210, image reconstruction is performed on the assumption that radiation imaging is performed at predetermined positions of coordinates (X1, Y₀, Z₀) and coordinates (X2, Y₀, Z₀). Therefore, the image actually obtained is corrected so as to become close to the radiation image to be obtained when radiation imaging is performed at the predetermined position.

In superimposed image G12' of image G1' and image G2' corrected in the manner described above, images 1Ag representing examination region 1A substantially overlap with each other. In this way, correction of each image obtained by tomosynthesis imaging prior to reconstruction may result in a high quality tomosynthesis image.

Note that a positional displacement in an X direction or in a Y direction can be corrected by shifting the image, and a positional displacement in a Z direction can be corrected by enlarging or reducing the image.

Hereinafter, a drawing illustrating tomosynthesis imaging, a drawing illustrating an error that occurs in tomosynthesis imaging, a drawing illustrating measurement results of acceleration sensors, and flowcharts of an acceleration measurement and tomosynthesis imaging will be described.

Figure 5 illustrates a positional relationship between each section when tomosynthesis imaging is performed.

As illustrated in Figure 5, the distance from tube focus 30F of radiation source 30 to examination region 1A is 1100 mm and the distance from examination region 1A to detection surface 20M of radiation detector 20 is 200 mm. The angle formed between the radiation emitted toward examination region 1A from first imaging position P1 and the radiation emitted toward examination region 1A from third imaging position P3 is 40°.

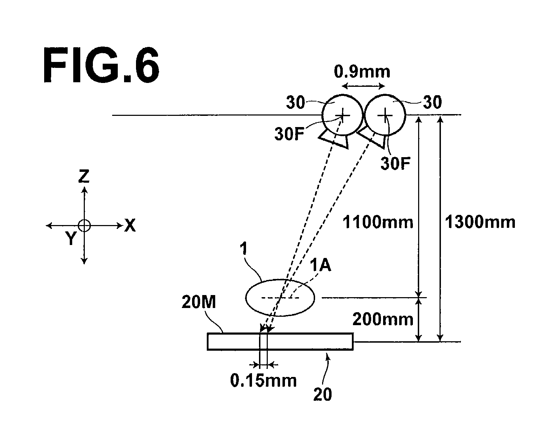

Figure 6 illustrates a tolerance when tomosynthesis imaging is performed with the positional relationship shown in Figure 5.

As illustrated in Figure 6, the pixel pitch of radiation detector 20 is 0.15 mm, so that the acceptable positional displacement of a radiation image formed on detection surface 20M is not greater than 0.15 mm. In order to limit the position error of a radiation image formed on the detection surface 20M to not greater than 0.15 mm when tomosynthesis imaging is performed with the positional relationship shown in Figure 5, it is necessary to limit the positional displacement of tube focus 30F in an X direction to not greater than 0.9 mm.

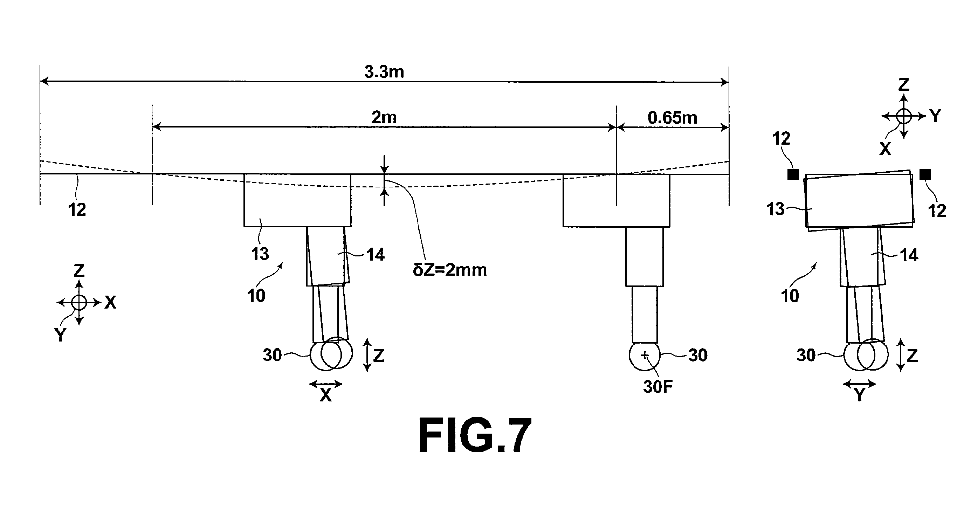

Figure 7 illustrates deflection of the movable rail extending in an X direction.

As illustrated in Figure 7, when shifting section 10, holding radiation source 30, is shifted along movable rail 12 extending in an X direction by 3.3 m, deflection δz of movable rail 12 reaches up to 2 mm. Further, along with the deflection, horizontal shifting platform 13 suspended by movable rail 12 is inclined in a Z-X plane, causing a positional displacement in an X direction. Still further, horizontal shifting platform 13 suspended by movable rail 12 is inclined in a Z-Y plane due to deformation of movable rail 12, causing a positional displacement in a Y direction.

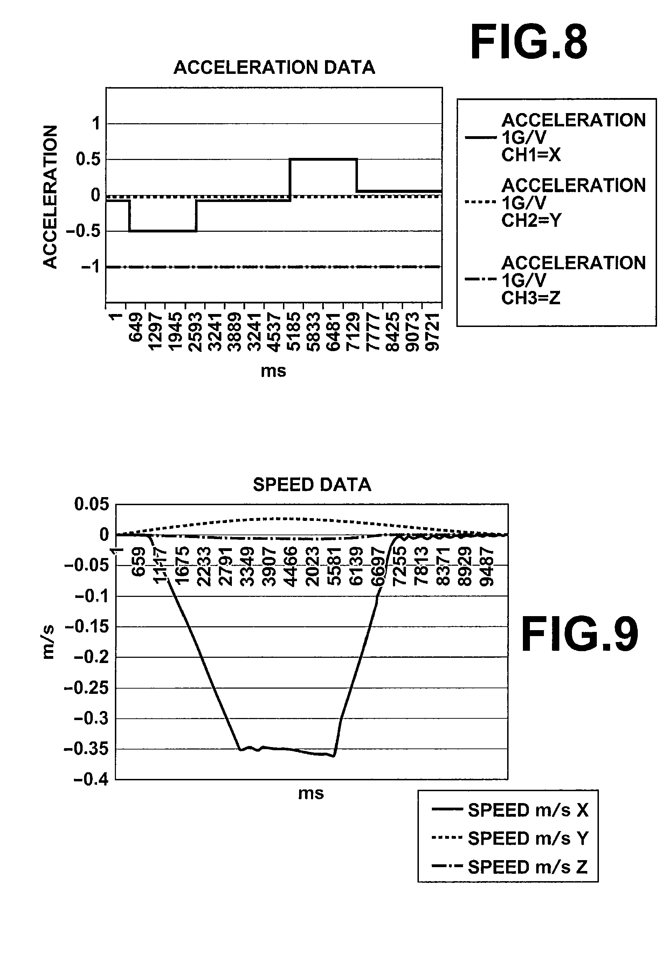

Figure 8 shows acceleration data measured by the acceleration sensor 40Sa (40Sb) when radiation source 30 is shifted in an X direction in a coordinate system with the vertical axis representing acceleration and the horizontal axis representing elapsed time. The reason why acceleration values in a Z direction remain constant at -1 is that the gravity is detected.

Figure 9 illustrates speed data obtained by integrating once the acceleration values measured by the acceleration sensor 40Sa (40Sb) when radiation source 30 is shifted in an X direction (Figure 8 above) in a coordinate system with the vertical axis representing speed and the horizontal axis representing elapsed time.

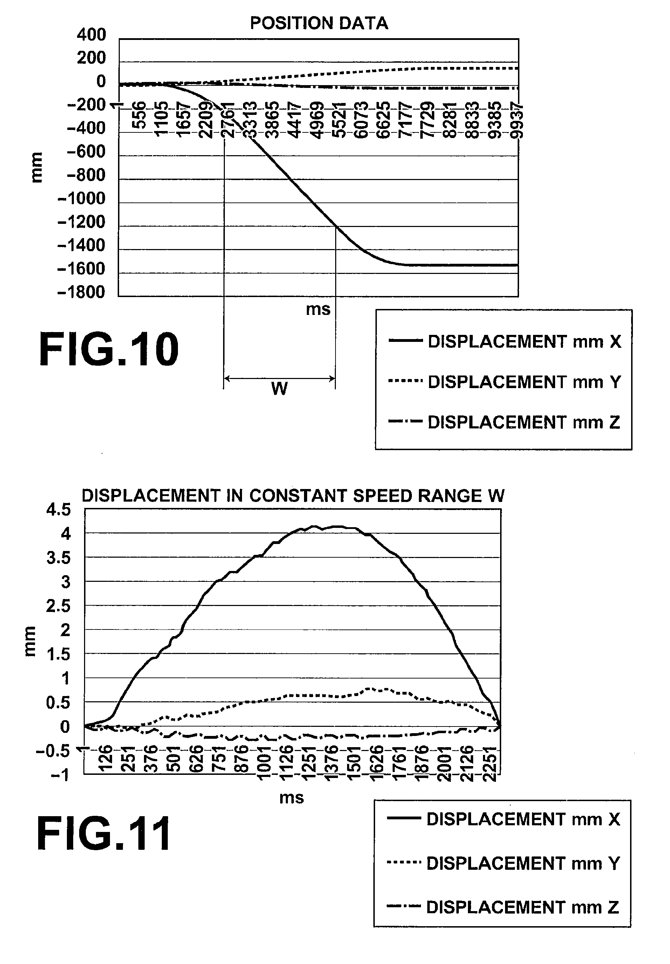

Figure 10 illustrates positions obtained by integrating the acceleration values twice measured by the acceleration sensor 40Sa (40Sb) when radiation source 30 is shifted in an X direction (Figure 8 above) in a coordinate system with the vertical axis representing position and the horizontal axis representing elapsed time.

It is known from Figures 8 to 10 that the amount of displacement from the reference position, i.e., the position of the tube focus may be measured only by the acceleration sensors attached to the radiation source.

Figure 11 shows position errors (displacements) of tube focus in a range in which horizontal shifting platform 13 is shifted at a constant speed (indicated by a reference symbol W in Figure 10).

As shown in Figure 11, the position error of tube focus in a Z direction (a vertical direction) and the position error of tube focus in a Y direction are not so large, but the position error (displacement from a predetermined position) in an X direction (a shifting direction) is large.

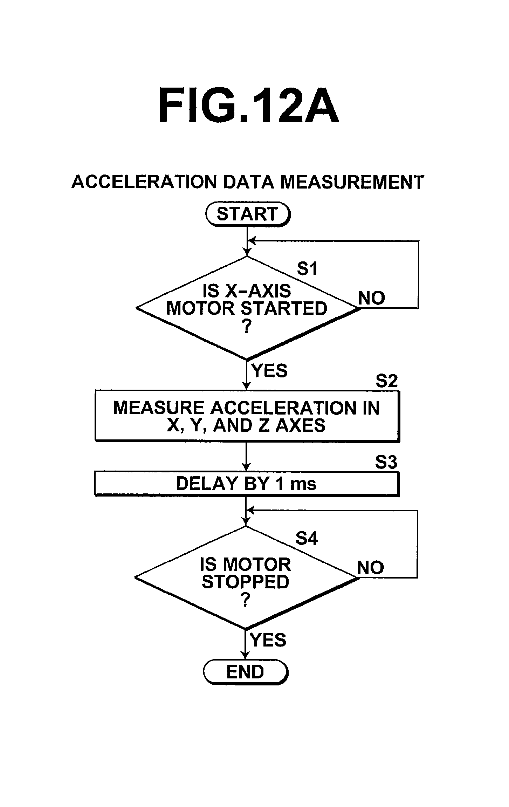

Figure 12A is a flowchart of an acceleration data measurement.

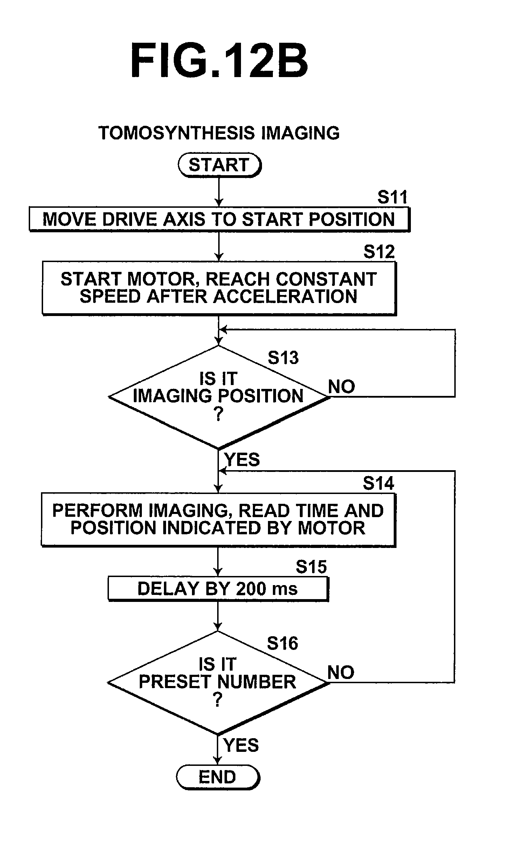

Figure 12B is a flowchart of tomosynthesis imaging.

The acceleration data measurement and tomosynthesis imaging are performed in the manner described in Figures 12A and 12B respectively.

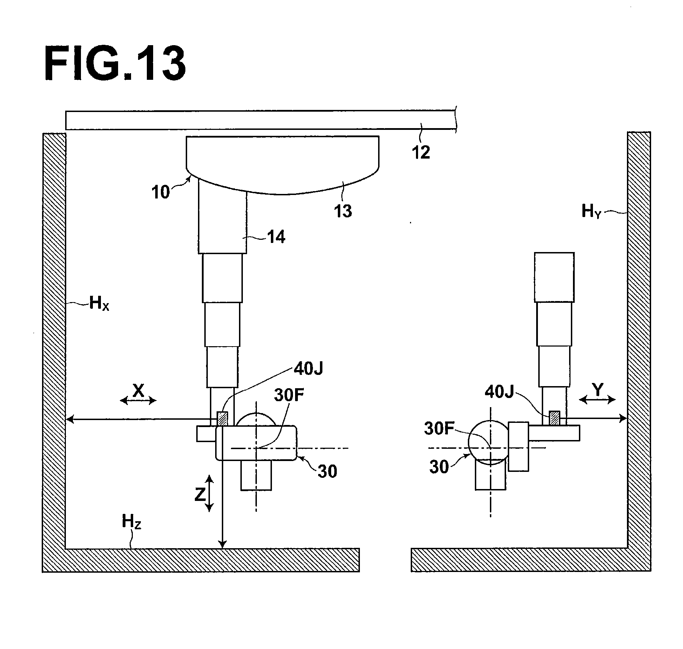

Hereinafter, a radiation imaging apparatus that uses a laser length measuring device for tube position measurement will be described. Figure 13 illustrates a front view and a side view of a radiation imaging apparatus that uses a laser length measuring device for tube position measurement. In Figure 13, the front view is shown on the left and the side view is shown on the right.

As illustrated in Figure 13, radiation imaging apparatus 300 that uses a laser length measuring device for tube position measurement performs the measurement by reflecting laser light on each of wall surface Hx serving as the measurement reference in an X direction, wall surface Hy serving as the measurement reference in a Y direction, and floor surface Hz serving as the measurement reference in a Z direction.

When tomosynthesis imaging by radiation imaging apparatus 300 is started, horizontal shifting platform 13 is shifted by shifting section 10 on movable rail 12 extending in an X direction. This causes radiation source 30, attached to the tip of extensible/retractable support 14, to be shifted also in the X direction, whereby the tube focus is also shifted in the X direction.

Laser light emitted from laser oscillator 40U attached to radiation source 30 passes through optical system 40J, serving as the base of laser length measurement, reflected on each of reference surfaces (wall surface Hx, wall surface Hy, floor surface Hz), and enters in optical system 40J again, whereby the position of tube focus 30F of radiation source 30 is measured.

Tomosynthesis imaging performed by radiation imaging apparatus 300 is substantially identical to the tomosynthesis imaging performed by radiation imaging apparatus described above.

Instead of using linear scale measuring devices 40L or laser oscillator 40U, a rotary encoder (not shown) may be attached to the rotary shaft of each of three drive motors, provided in shifting section 10, for shifting radiation source in three axis directions in order to measure the position of tube focus in three axis directions.

## Claims

1. A radiation imaging apparatus which includes a radiation source (30) for emitting radiation, a radiation detector (20) for detecting radiation emitted from the radiation source (30) and transmitted through a subject (1A), a shifting means (10) for holding and shifting the radiation source (30) in a direction parallel to a detection surface (20M) of the radiation detector (20), and a position obtaining means (40) for obtaining a position of a tube focus (30F) of the radiation source (30), and which performs tomosynthesis imaging while shifting the radiation source (30) by the shifting means (10),
wherein the position obtaining means comprises an acceleration sensor (40S) integrally disposed adjacent to the radiation source (30).

2. The radiation imaging apparatus of claim 1, **characterized in that** the acceleration sensor (40S) comprises a pair of acceleration sensors (40Sa, 40Sb) disposed on a straight line passing through the tube focus (30F) so as to sandwich the tube focus (30F).

3. The radiation imaging apparatus of claim 1 or 2, **characterized in that** the shifting means (10) is a means that includes an orthogonal shifting section for shifting the radiation source (30) in a direction orthogonal to the detection surface (20M).

4. The radiation imaging apparatus of any of claims 1 to 3, **characterized in that** the radiation source (30) is a source held by an overhead traveling suspension shifting platform (13).

5. A radiation imaging apparatus which includes a radiation source (30) for emitting radiation, a radiation detector (20) for detecting radiation emitted from the radiation source (30) and transmitted through a subject (1A), a shifting means (10) for holding and shifting the radiation source (30) in a direction parallel to a detection surface (20M) of the radiation detector (20), and a position obtaining means (40) for obtaining a position of a tube focus (30F) of the radiation source (30), and which performs radiation imaging while shifting the radiation source (30) by the shifting means (10),
wherein the position obtaining means (40) comprises a laser length measuring device integrally disposed adjacent to the radiation source (30).
